(19) Europäisches Patentamt
European Patent Office
Office européen des brevets

(11) EP 1 277 771 A2

(12) **DEMANDE DE BREVET EUROPEEN**

(43) Date de publication:
**22.01.2003 Bulletin 2003/04**

(51) Int Cl.[7]: **C08F 293/00**, C07C 255/65, C08F 2/50

(21) Numéro de dépôt: **02078512.7**

(22) Date de dépôt: **22.10.1998**

(84) Etats contractants désignés:
**AT BE DE ES FI FR GB IT NL SE**

(30) Priorité: **24.10.1997 FR 9713383**

(62) Numéro(s) de document de la (des) demande(s) initiale(s) en application de l'article 76 CBE:
**98402624.5 / 0 911 350**

(71) Demandeur: **Atofina**
**92800 Puteaux (FR)**

(72) Inventeurs:
- **Bertin Denis**
**76970 Motteville (FR)**
- **Destarac Mathias**
**30220 Aigue-Mortes (FR)**
- **Boutevin Bernard**
**34090 Montpellier (FR)**

(74) Mandataire: **Chaillot, Geneviève**
**Cabinet CHAILLOT,**
**16-20, avenue de L'Agent Sarre,**
**B.P. 74**
**92703 Colombes Cédex (FR)**

Remarques:
Cette demande a été déposée le 26 - 08 - 2002 comme demande divisionnaire de la demande mentionnée sous le code INID 62.

(54) **Procédé de fabrication d'un polymère séquencé à l'aide d'amorceurs radicalaires fonctionnels en polymérisation vivante**

(57) Dans une première étape, on conduit une polymérisation radicalaire par voie thermique d'au moins un monomère $M_1$ à l'aide d'un amorceur de formule (I) X-A-Y dans laquelle : X et Y représentent chacun un groupement fonctionnel monovalent réactif et réactivable en polymérisation radicalaire vivante ; A représente un groupement divalent comprenant une liaison capable, en polymérisation radicalaire de se diviser pour générer des radicaux libres X-D* et *E-Y, D et E étant les deux fragments de A issus de la division de ladite liaison, pour obtenir un polymère pouvant être représenté par la formule (II) X-D-(ou Y-E-)$PM_1$-T où T représente (1) un groupe terminal X-D- ou Y-E- qui se forme par recombinaison et/ou (2) un groupe terminal qui comporte une liaison insaturée et une liaison saturée et qui se forme par dismutation, les terminaisons T étant totalement ou essentiellement de type (2) dans le cas où M1 est un monomère méthacrylique ; dans une seconde étape, on conduit une polymérisation radicalaire vivante du polymère (II) ainsi obtenu, conduisant à l'insertion entre les X et D et entre les Y et E de ce dernier d'une séquence polymère $PM_2$ d'au moins un monomère $M_2$, le copolymère vivant obtenu pouvant être utilisé comme macroamorceur dans une nouvelle étape de polymérisation radicalaire vivante d'au moins un monomère $M_3$, avec la condition que $PM_2$ ne représente pas une séquence de polystyrène ou de copolymère styrène-acrylonitrile (a) pour une séquence $PM_1$ (méth) acrylique ou de polyisoprène, (b) dans le cas de copolymères triséquencés, (c) lesquels ont été obtenus avec un amorceur (I) de type azoïque, (d) dans lequel X et Y représentent des groupements donnant des radicaux 2,2,6,6-tétraméthyl-1-pipéridinyloxy en copolymérisation radicalaire vivante.



Printed by Jouve, 75001 PARIS (FR)

## Description

La présente invention concerne un procédé de fabrication de copolymères à architecture contrôlée à l'aide d'amorceurs radicalaires fonctionnels en polymérisation radicalaire vivante. Les copolymères obtenus peuvent être des polymères biséquencés, triséquencés, voire comportant un plus grand nombre de séquences.

Ce procédé combine la polymérisation radicalaire classique (désignée dans ce qui suit simplement par polymérisation radicalaire) et la polymérisation radicalaire vivante. Dans Macromolecules, Vol. 30, N° 18, 8 septembre 1997, pages 5195-5199, I.Q. Li et al. décrivent la préparation d'un amorceur azoïque terminé à chaque extrémité par des radicaux nitroxyde, cet amorceur étant utilisé pour la polymérisation radicalaire classique d'acrylate de n-butyle, de méthacrylate de méthyle et d'isoprène, le macroamorceur terminé par les radicaux nitroxyde servant à la copolymérisation radicalaire vivante du styrène ou du styrène et de l'acrylonitrile. Les brevets américains US-A-5 627 248 et US-A-5 667 388 décrivent d'une manière plus générale une telle préparation en citant également les amorceurs de type dinitroxyl disulfure et dinitroxyl peroxyde, ainsi que la copolymérisation radicalaire classique du butadiène.

La Société déposante propose d'utiliser d'autres voies de polymérisation radicalaire vivante et/ou d'autres types d'amorceurs, permettant de proposer de nouveaux copolymères séquencés.

La présente invention a donc pour objet un procédé de fabrication d'un copolymère séquencé, caractérisé par le fait que :

- dans une première étape, on conduit une polymérisation radicalaire par voie thermique d'au moins un monomère $M_1$ à l'aide d'un amorceur de formule (I) :

$$X\text{-}A\text{-}Y \qquad (I)$$

  dans laquelle :
- X et Y représentent chacun un groupement fonctionnel monovalent réactif et réactivable en polymérisation radicalaire vivante ;
- A représente un groupement divalent comprenant une liaison capable, en polymérisation radicalaire de se diviser pour générer des radicaux libres $X\text{-}D^{\bullet}$ et $^{\bullet}E\text{-}Y$, D et E étant les deux fragments de A issus de la division de ladite liaison,
  pour obtenir un polymère pouvant être représenté par la formule (II) :

$$X\text{-}D\text{-}(\text{ou } Y\text{-}E\text{-})PM_1\text{-}T \qquad (II)$$

  où T représente (1) un groupe terminal X-D- ou Y-E- qui se forme par recombinaison et/ou (2) un groupe terminal qui comporte une liaison insaturée et une liaison saturée et qui se forme par dismutation, les terminaisons T étant totalement ou essentiellement de type (2) dans le cas où $M_1$ est un monomère méthacrylique ;
- dans une seconde étape, on conduit une polymérisation radicalaire vivante du polymère (II) ainsi obtenu, conduisant à l'insertion entre les X et D et entre les Y et E de ce dernier d'une séquence polymère $PM_2$ d'au moins un monomère $M_2$, le copolymère vivant obtenu pouvant être utilisé comme macroamorceur dans une nouvelle étape de polymérisation radicalaire vivante d'au moins un monomère $M_3$,

avec la condition que $PM_2$ ne représente pas une séquence de polystyrène ou de copolymère styrène-acrylonitrile

(a) pour une séquence $PM_1$ (méth)acrylique ou de polyisoprène
(b) dans le cas de copolymères triséquencés
(c) lesquels ont été obtenus avec un amorceur (I) de type azoïque
(d) dans lequel X et Y représentent des groupements donnant des radicaux 2,2,6,6-tétraméthyl-1-pipéridinyloxy en copolymérisation radicalaire vivante.

On peut prévoir que $PM_2$ ne représente pas une séquence d'un copolymère obtenu à partir d'un monomère vinylaromatique dans les conditions (a), (b), (c) et (d) telles qu'indiquées ci-dessus.

On peut également prévoir que la condition (a) s'applique aussi pour une séquence $PM_1$ de polybutadiène, et, d'une manière plus générale, qu'elle s'applique aux séquences (méth)acryliques ou diéniques.

On peut aussi prévoir qu'indépendamment :

- la condition (b) s'applique aussi aux copolymères biséquencés ;
- la condition (c) s'applique aussi aux amorceurs (I) de type disulfure ou peroxyde ; et
- la condition (d) s'applique de manière générale au cas où X et Y représentent des groupements donnant des radicaux nitroxyde en copolymérisation radicalaire vivante.

La présente invention concerne aussi un procédé de fabrication d'un polymère séquencé, caractérisé par le fait que :

- dans une première étape, on conduit une polymérisation radicalaire par voie thermique d'au moins un monomère $M_1$ à l'aide d'un amorceur de formule (I) :

$$X\text{-}A\text{-}Y \qquad (I)$$

dans laquelle :

- X et Y représentent chacun un groupement fonctionnel monovalent réactif et réactivable en polymérisation radicalaire vivante ;
- A représente un groupement divalent comprenant une liaison capable, en polymérisation radicalaire de se diviser pour générer des radicaux libres $X\text{-}D^{\bullet}$ et $^{\bullet}E\text{-}Y$, D et E étant les deux fragments de A issus de la division de ladite liaison,
  pour obtenir un polymère pouvant être représenté par la formule (II) :

$$X\text{-}D\text{-}(ou\ Y\text{-}E\text{-})PM_1\text{-}T \qquad (II)$$

  où T représente (1) un groupe terminal X-D- ou Y-E- qui se forme par recombinaison et/ou (2) un groupe terminal comporte une liaison insaturée et une liaison saturée et qui se forme par dismutation, les terminaisons T étant totalement ou essentiellement de type (2) dans le cas où $M_1$ est un monomère méthacrylique ;
- dans une seconde étape, on conduit une polymérisation radicalaire vivante du polymère (II) ainsi obtenu, ladite polymérisation radicalaire vivante étant une polymérisation à activation photochimique en présence de radicaux libres ou une polymérisation contrôlée par transfert d'atomes en présence de radicaux libres, et conduisant à l'insertion entre les X et D et entre les Y et E de ce dernier d'une séquence polymère $PM_2$ d'au moins un monomère $M_2$, le copolymère vivant obtenu pouvant être utilisé comme macroamorceur dans une nouvelle étape de polymérisation radicalaire vivante d'au moins un monomère $M_3$.

Les monomères $M_1$ et $M_2$ et le cas échéant $M_3$ sont notamment choisis parmi les monomères vinyliques, allyliques, vinylidéniques, diéniques et oléfiniques.

Par monomères vinyliques, on entend les (méth)acrylates, les monomères vinylaromatiques, les esters vinyliques, les éthers vinyliques, le (méth)acrylonitrile, le (méth)acrylamide et les mono- et di-(alkyl en $C_1$-$C_{18}$)-(méth) acrylamides, et les monoesters et diesters de l'anhydride maléique et de l'acide maléique.

- Les (méth)acrylates sont en particulier ceux des formules respectivement :

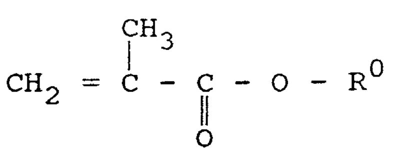 et 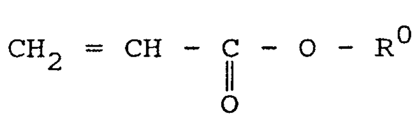

dans lesquelles $R^0$ est choisi parmi les radicaux alkyle en $C_1$-$C_{18}$, linéaires ou ramifiés, primaires, secondaires ou tertiaires, cycloalkyle en $C_5$-$C_{18}$, (alcoxy en $C_1$-$C_{18}$)-alkyle en $C_1$-$C_{18}$, (alkylthio en $C_1$-$C_{18}$)-alkyle en $C_1$-$C_{18}$, aryle et arylalkyle, ces radicaux étant éventuellement substitués par au moins un atome d'halogène et/ou au moins un groupe hydroxyle, les groupes alkyle ci-dessus étant linéaires ou ramifiés ; et les (méth)acrylates de glycidyle, de norbornyle, d'isobornyle.

Comme exemples de méthacrylates utiles, on peut citer les méthacrylates de méthyle, d'éthyle, de 2,2,2-trifluoroéthyle, de n-propyle, d'isopropyle, de n-butyle, de sec.-butyle, de tert.-butyle, de n-amyle, d'i-amyle, de n-hexyle, de 2-éthylhexyle, de cyclohexyle, d'octyle, d'ioctyle, de nonyle, de décyle, de lauryle, de stéaryle, de phényle, de benzyle, de β-hydroxy-éthyle, d'isobornyle, d'hydroxypropyle, d'hydroxybutyle.

Comme exemples d'acrylates de la formule ci-dessus, on peut citer les acrylates de méthyle, d'éthyle, de n-propyle, d'isopropyle, de n-butyle, de sec.-butyle, de tert.-butyle, d'hexyle, de 2-éthylhexyle, d'isooctyle, de 3,3,5-triméthylhexyle, de nonyle, d'isodécyle, de lauryle, d'octadécyle, de cyclohexyle, de phényle, de méthoxyméthyle, de méthoxyéthyle, d'éthoxyméthyle et d'éthoxyéthyle.

- Par monomère vinylaromatique au sens de la présente invention, on entend un monomère aromatique à insaturation éthylénique tel que le styrène, le vinyltoluène, l'alphaméthylstyrène, le méthyl-4-styrène, le méthyl-3-styrène, le méthoxy-4-styrène, l'hydroxyméthyl-2-styrène, l'éthyl-4-styrène, l'éthoxy-4-styrène, le diméthyl-3,4-styrène, le chloro-2-styrène, le chloro-3-styrène, le chloro-4-méthyl-3-styrène, le tert.-butyl-3-styrène, le dichloro-2,4-styrène, le dichloro-2,6-styrène et le vinyl-1-naphtalène.
- Comme esters vinyliques, on peut citer l'acétate de vinyle, le propionate de vinyle, le chlorure de vinyle et le fluorure de vinyle, et comme éthers vinyliques, on peut citer le vinyl méthyl éther et le vinyl éthyl éther.

Comme monomère vinylidénique, on cite le fluorure de vinylidène.

Par monomère diénique, on entend un diène choisi parmi les diènes linéaires ou cycliques, conjugués ou non-conjugués comme par exemple le butadiène, le 2,3-diméthylbutadiène, l'isoprène, le 1,3-pentadiène, le 1,4-pentadiène, le 1,4-hexadiène, le 1,5-hexadiène, le 1,9-décadiène, le 5-méthylène-2-norbornène, le 5-vinyl-2-norbornène, les 2-alkyl-2,5-norbornadiènes, le 5-éthylène-2-norbornène, le 5-(2-propényl)-2-norbornène, le 5-(5-hexényl)-2-norbornène, le 1,5-cyclooctadiène, le bicyclo[2,2,2]octa-2,5-diène, le cyclopentadiène, le 4,7,8,9-tétrahydroindène et l'isopropylidène tétrahydroindène.

Comme monomères oléfiniques, on peut citer l'éthylène, le butène, l'hexène et le 1-octène. Les monomères oléfiniques fluorés peuvent également être cités.

A comporte notamment un groupement choisi parmi les groupements : -N=N-, -O-O-,

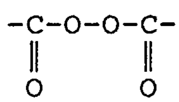,
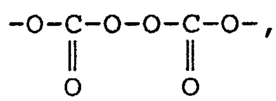,

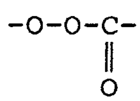 et 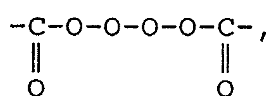,

dont le clivage fournit les radicaux libres en polymérisation radicalaire.

Un exemple de l'amorceur (I) est celui issu de la réaction de l'acide 4,4'-azobis(4-cyano-pentanoïque) avec un alcool portant un groupement fonctionnel X ou Y. Un autre exemple de l'amorceur (I) est celui issu de la réaction entre le peroxyde d'hydrogène et un chlorure d'acide portant un groupement X ou Y.

On conduit généralement la polymérisation de la première étape à une température comprise dans une plage allant de 0°C à une température inférieure à la température de dissociation des groupements X et Y, en particulier à une température de 20 à 200°C, excepté dans le cas où X et Y donnent des radicaux nitroxyde à la deuxième étape, où cette plage de température est de 20 à 100°C. En effet, au-dessus de 100°C, la liaison C-nitroxyde est labile.

Conformément à un premier mode de réalisation, la polymérisation radicalaire vivante de la deuxième étape est une polymérisation à activation thermique en présence de radicaux libres stables comprenant le groupement =N-O•, les groupements X et Y représentant alors chacun notamment un reste choisi parmi ceux des formules (IIIa) et (IIIb) :

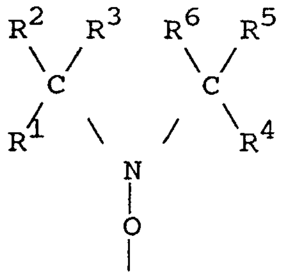

(IIIa)

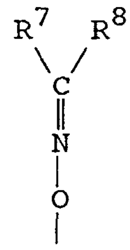

(IIIb)

où :

- $R^1$, $R^2$, $R^3$, $R^4$, $R^5$, $R^6$, $R^7$ et $R^8$, identiques ou différents, représentent chacun :
  - un atome d'halogène, tel que le chlore, le brome ou l'iode ;
  - un groupement hydrocarboné linéaire, ramifié ou cyclique, saturé ou insaturé, tel qu'un radical alkyle ou phényle ;
  - un groupement ester $-COOR^9$ ou un groupement alcoxyle $-OR^{10}$ ou un groupement phosphonate $-PO(OR^{11})_2$ où $R^9$, $R^{10}$ et les $R^{11}$ représentent chacun indépendamment un groupement hydrocarboné linéaire, ramifié ou cyclique, saturé ou insaturé ;
  - une chaîne de polymère pouvant être par exemple une chaîne de poly(méthacrylate de méthyle), de polybutadiène, de polyoxyéthylène, de polyoxypropylène, de polyoléfine comme de polyéthylène ou de polypropylène mais étant, de préférence, une chaîne de polystyrène,

  $R^3$ et $R^6$ pouvant être reliés entre eux pour former un groupement

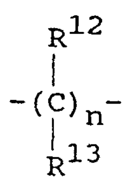

;

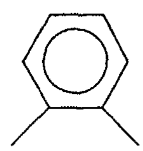

;

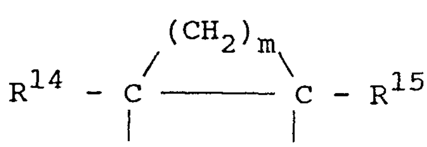

;

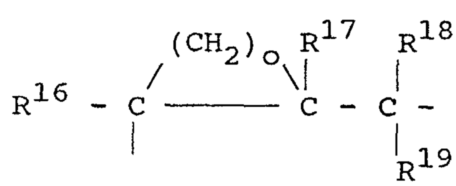

où $R^{12}$, $R^{13}$, $R^{14}$, $R^{15}$, $R^{16}$, $R^{17}$, $R^{18}$ et $R^{19}$ représentent chacun indépendamment un atome d'hydrogène, OH, -COOH, $-PO(OH)_2$, $-SO_3H$ ou ont une signification choisie parmi celles envisagées ci-dessus pour $R^1$ à $R^8$ ;
- n vaut 2 ou 3, les $R^{12}$ et les $R^{13}$ portés par les différents atomes de carbone pouvant être identiques ou différents ;
- m et o représentent chacun un entier de 1 à 10, ladite polymérisation radicalaire vivante étant conduite à une température supérieure à 80°C.

Le radical libre stable est choisi en particulier parmi :

- le 2,2,5,5-tétraméthyl-1-pyrrolidinyloxy (généralement commercialisé sous la marque PROXYL) ;
- le 2,2,6,6-tétraméthyl-1-pipéridinyloxy (généralement commercialisé sous la dénomination TEMPO) ;
- le N-tertiobutyl-1-phényl-2-méthyl propyl nitroxyde ;

- le N-tertiobutyl-1-(2-naphtyl)-2-méthyl propyl nitroxyde ;
- le N-tertiobutyl-1-diéthylphosphono-2,2-diméthyl propyl nitroxyde ;
- le N-tertiobutyl-l-dibenzylphosphono-2,2-diméthyl propyl nitroxyde,
- le N-phényl-l-diéthylphosphono-2,2-diméthyl propyl nitroxyde ;
- le N-(1-phényl 2-méthyl propyl)-1-diéthylphosphono-1-méthyl éthyl nitroxyde.

Conformément à un deuxième mode de réalisation de la présente invention, la polymérisation radicalaire vivante de la deuxième étape est une polymérisation à activation photochimique en présence de radicaux libres, en particulier de radicaux libres provenant de groupements X et Y de formule (IV) :

$$R^{20}\text{-S-} \qquad (IV)$$

où $R^{20}$ est choisi parmi les radicaux alkyle, aryle, aralkyle et alkaryle, ayant par exemple 1 à 30 atomes de carbone et pouvant comporter des substituants ; et les radicaux des formules :

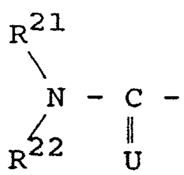 ; 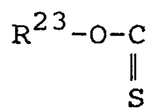

où U représente O ou S, et $R^{21}$, $R^{22}$ et $R^{23}$ sont choisis chacun parmi les radicaux alkyle, aryle, aralkyle et alkaryle, ayant par exemple de 1 à 30 atomes de carbone et pouvant comporter des substituants, $R^{21}$ et $R^{22}$ pouvant former, conjointement avec l'atome d'azote qui les porte, un cycle azoté en $C_4$ -$C_{10}$, pouvant comporter des substituants.

Des exemples de groupements X et Y sont dans ce cas :

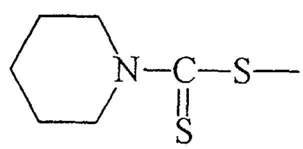 ; 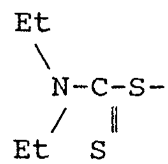 ;

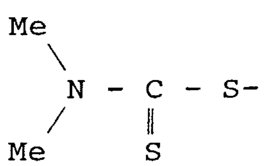 ; 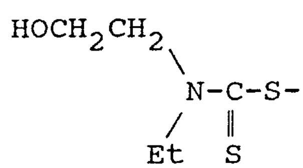 ;

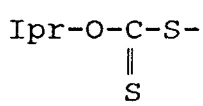

ou le radical N-(2-phtalimido éthyl)pipérazine thiurame.

On peut également se rapporter aux brevets européens EP 507 036, EP 418 118, EP 342 073 et EP 338 918 pour compléter cette liste d'exemples.

Ladite photopolymérisation est généralement conduite en présence d'un rayonnement UV à une température comprise entre -50°C et 100°C.

Conformément à un troisième mode de réalisation de la présente invention, la polymérisation radicalaire vivante de la deuxième étape est une polymérisation contrôlée par transfert d'atomes, en présence de radicaux libres, en particulier de radicaux libres provenant de X et Y qui représentent chacun un groupement choisi parmi :

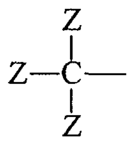 ; 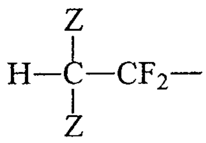 ; 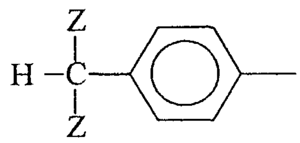 ; 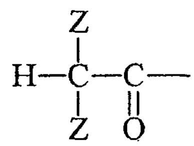 ;

(a) (b) (c) (d)

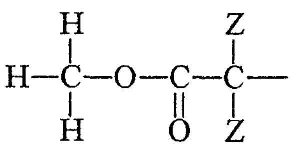 ; 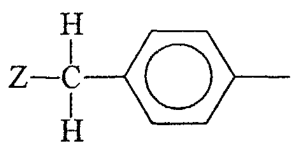 ; 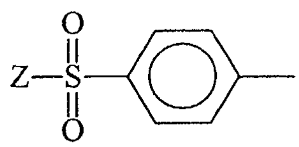 ;

(e) (f) (g)

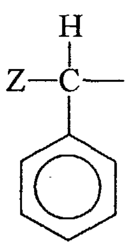 ; 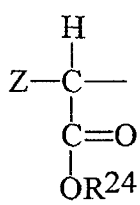 ; 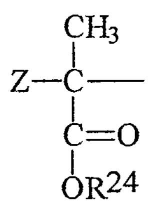 ; 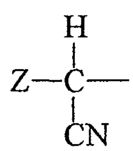 ;

(h) (i) (j) (k)

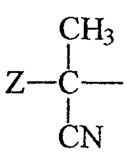 ; 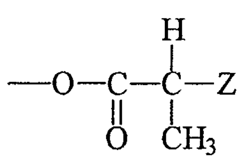 ; 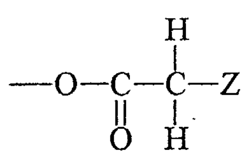

(l) (m) (n)

où Z représente chlore ou brome et $R^{24}$ représente hydrogène, alkyle ou aryle, ladite réaction de polymérisation pouvant être catalysée par des complexes de métaux de transition, tels que :

- les complexes de cuivre de type CuZ'/L, où :
  - Z' représente un atome d'halogène ou encore l'ion hexafluorophosphate $PF_6^-$ ou un groupement acétate ; et
  - L est un ligand $\alpha$-diimine, de squelette -N=C-C=N-, cette famille incluant la 2,2'-bipyridine et ses dérivés, la 1,10-phénanthroline et ses dérivés, les 1,4-diazo-1,3-butadiènes et les 2-pyridinecarbaldéhyde imines de structures :

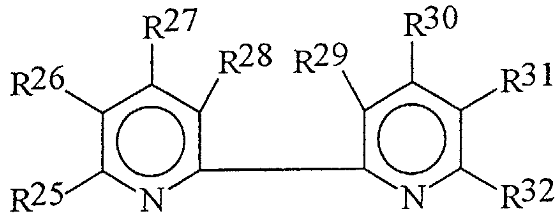

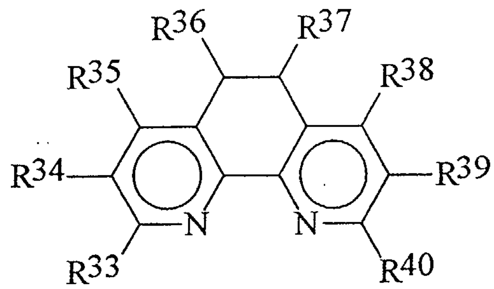

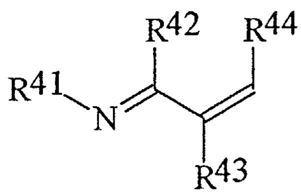

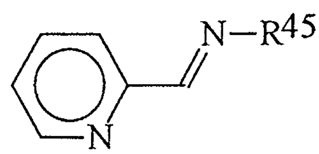

où $R^{25}$ à $R^{45}$ représentent chacun hydrogène, alkyle ou aryle ; et

- les catalyseurs complexes des métaux Ni, Pd ou Pt (tels que ceux décrits dans la demande européenne EP-97 401 440 du 20 juin 1997) ; Fe, Ru ou Os (tels que ceux décrits dans la demande européenne EP-97 401 441 du 20 juin 1997) ; Pd (tels que ceux décrits dans la demande européenne EP-97 400 600.9 du 18 mars 1997) ; Rh, Co ou Ir (tels que ceux décrits dans la demande européenne EP-97 401 442.5 du 20 juin 1997) ; et Cu, Ag

  ou Au (tels que ceux décrits dans la demande européenne EP-97 401 443.3 du 20 juin 1997).

La présente invention porte également sur les copolymères susceptibles d'être obtenus par le procédé qui vient d'être décrit, ainsi que sur les composés de formule (V) :

$$X - L_1 - A' - L_2 - Y \qquad (V)$$

dans laquelle :

- A' représente un groupement choisi parmi les groupements: -N=N-, -O-O-,

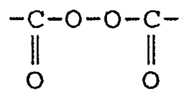,
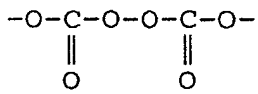,

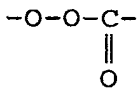 et 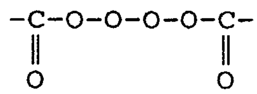 ;

- $L_1$ et $L_2$ représentent chacun un groupement de liaison divalent ; et
- X et Y représentent chacun un groupement choisi parmi ceux des formules (IIIa) et (IIIb) ci-dessus ; ceux de la formule (IV) ci-dessus ; et les groupements :

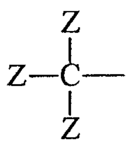
(a) ;

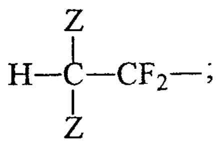
(b) ;

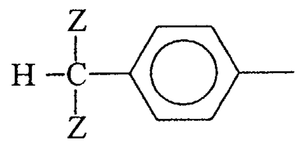
(c) ;

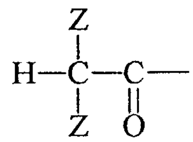
(d) ;

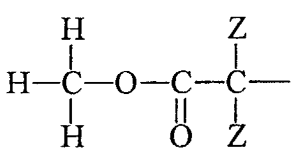
(e) ;

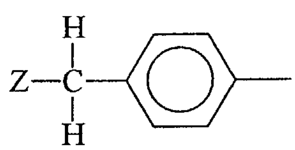
(f) ;

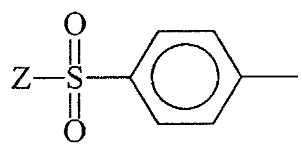
(g) ;

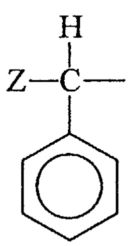
(h) ;

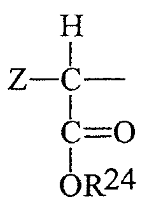
(i) ;

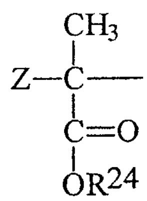
(j) ;

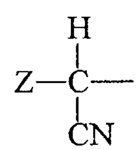
(k) ;

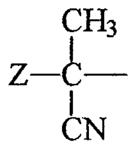 ; 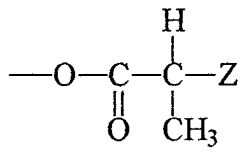 ; 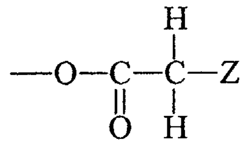

(l) (m) (n)

où :

- Z représente chlore ou brome ; et
- $R^{24}$ représente hydrogène, alkyle ou aryle, X et Y ne pouvant représenter un groupement 2,2,6,6-tétraméthyl-1-pipéridinyloxy lorsque A représente -N=N-.

X et Y pourraient aussi ne pas représenter un groupement de formule (IIIa) lorsque A' représente -N=N-.

Egalement, X et Y pourraient ne pas représenter un groupement 2,2,6,6-tétraméthyl-1-pipéridinyloxy ou de façon plus générale un groupement de formule (IIIa) lorsque A' représente

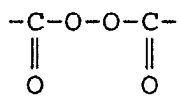 ou 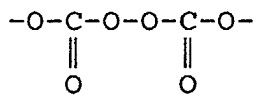 .

La présente invention concerne aussi un composé de formule (V) dans laquelle A' représente un groupement choisi parmi les groupements: -O-O-,

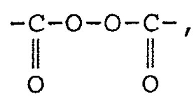 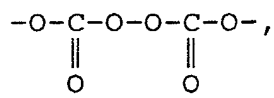

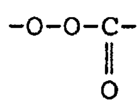 et 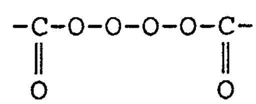 ;

$L_1$ et $L_2$ et X et Y ont les significations indiquées ci-dessus, A' pouvant également représenter -N=N- lorsque X et Y représentent un groupement choisi parmi ceux des formules (IIIb) et (IV) et (a) à (n) telles que définies ci-dessus.

On peut citer les composés pour lesquels A' représente -O-O-,

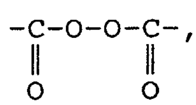 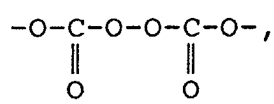 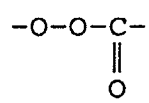 et

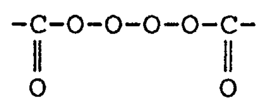 ;

et X et Y sont ceux représentés par les formules (IIIa), (IIIb) et (IV) et (a) à (n) ci-dessus ; et ceux pour lesquels A' représente le groupement -N=N- et X et Y sont choisis parmi ceux de formule (IIIb), (IV) et (a) à (n) ci-dessus.

On peut citer en particulier les composés :

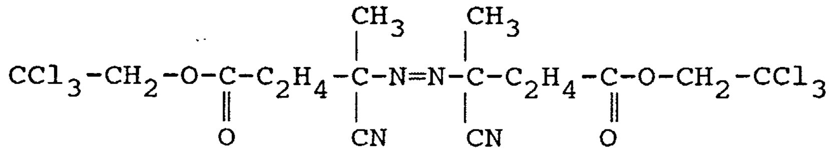

;

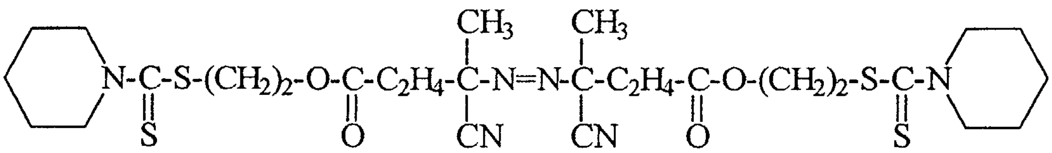

; et

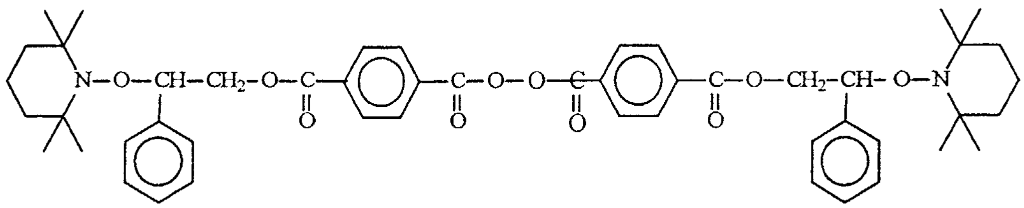

Les Exemples suivants illustrent la présente invention sans toutefois en limiter la portée. Dans ces exemples, on a utilisé les abréviations suivantes :

- THF : tétrahydrofuranne
- ACP : acide 4,4'-azobis(4-cyano-pentanoique)

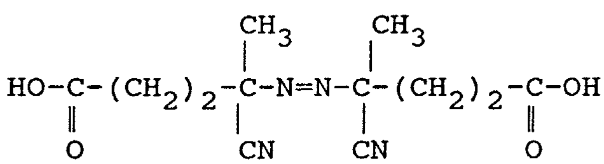

- DCC : dicyclohexylcarbodiimide

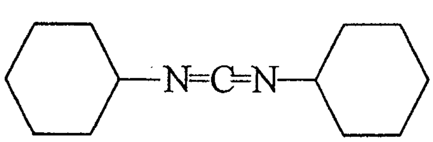

- DMAP :

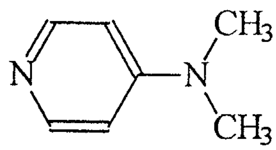

- DCU : dicyclohexylurée

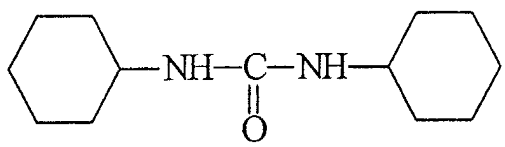

- ABu : acrylate de n-butyle
- PABu : séquence de poly(acrylate de n-butyle)
- S : styrène
- PS : séquence de polystyrène
- MMA : méthacrylate de méthyle
- PMMA : séquence de poly(méthacrylate de méthyle)
- PB : polybutadiène

  $\overline{Mn}$ : Masse moléculaire moyenne en nombre

  $\overline{DP_n}$ : Degré de polymérisation moyen en nombre

EXEMPLE 1 : Préparation d'un polymère triséquencé PS-PABu-PS

(a) Synthèse de l'amorceur :

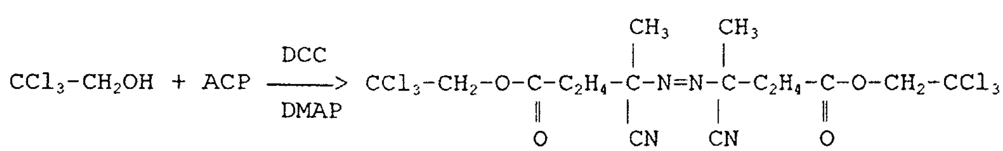


(Lors de la réaction, la DCC donne la DCU qu'il y aura lieu d'éliminer)

• Mode opératoire

Une solution de 11,18 g d'ACP (39,9 mmoles), 13,70 g de trichloroéthanol (92,2 mmoles) et 250 ml de THF est homogénéisée dans un ballon de 500 ml.

A refroidissement à 0°C, on ajoute, par une ampoule à brome, une solution contenant 19,02 g de DCC (92,2 mmoles), 0,5 g de DMAP (4,1 mmoles) et 50 ml de THF.

Après 10 minutes environ, on observe l'apparition d'un précipité blanc de DCU.

Après 40 minutes, l'addition est terminée et on laisse la réaction se poursuivre à la température ambiante.

La solution obtenue est filtrée sur fritté n° 5 pour éliminer la DCU. Enfin, on évapore à froid pour ne pas décomposer l'amorceur. On obtient une huile que l'on reprend par 100 ml de chloroforme. Cette solution est précipitée lentement dans 1,5 l de pentane à 0°C. On filtre ensuite pour récupérer l'amorceur précipité. On le sèche sous 1 x $10^{-3}$ bar. On récupère 6,35 g de poudre blanche.

RMN $^1$H (Bruker, 200 MHz) (δ) :

4,75 (s, $C\underline{H_2}CCl_3$) ;
2,5 (m, $CO\text{-}C_2\underline{H_4}\text{-}C$) ;
1,75 (s, $C\underline{H_3}$).

(b) Première étape de la polymérisation

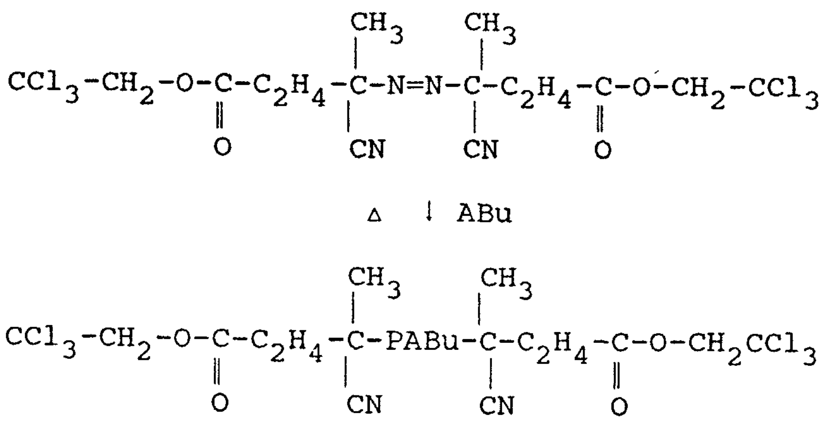


250 mg de cet amorceur sont solubilisés dans 3,93 g d'ABu. Dans un ballon bicol de 50 ml surmonté d'un réfrigérant, 13,1 ml de cyclohexanone sont portés à 130°C. Après trois cycles de purge (vide-azote), le système est placé sous atmosphère inerte et le mélange monomère-amorceur est ajouté directement dans la cyclohexanone à 130°C, le tout sous agitation magnétique. Après quatre minutes, le chauffage est arrêté et le ballon est plongé dans un bain de glace afin de stopper la polymérisation.

Après évaporation du solvant (traitement thermique sous vide), la conversion massique du monomère est de 80%.

Par analyse RMN $^1$H du PABu, la masse moléculaire moyenne en nombre ($\overline{Mn}$) a été déterminée en calculant le rapport des hauteurs des pics correspondant aux protons du PABu et des protons en alpha du $CCl_3$. La $\overline{Mn}$ obtenue est de 8700 g/mole, soit un $\overline{DP}_n$ de 64.

Par GPC dans le THF, avec un étalonnage de polystyrènes standards, la $\overline{Mn}$ est de 3100 g/mole.

(c) Deuxième étape de la polymérisation

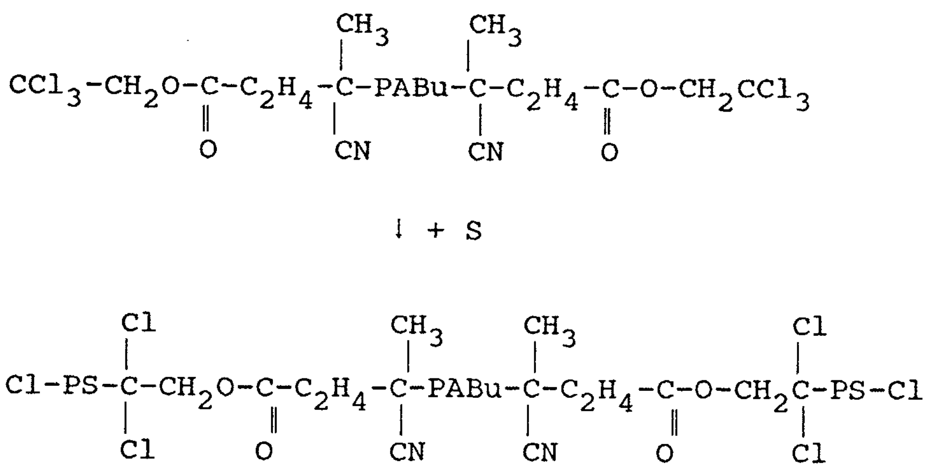


Dans un ballon monocol de 50 ml surmonté d'un take-off et d'un robinet, 1 g du PABu de 8700 g/mole précédemment synthétisé, 20,83 g de styrène, 500 mg de CuCl et 2,36 g de bipyridine sont ajoutés.

Le mélange réactionnel est immédiatement dégazé sous vide selon les conditions opératoires suivantes :

1 - Solidification du mélange en plongeant le ballon dans de l'azote liquide ;
2 - Mise sous vide ;
3 - Liquéfaction du mélange en le plongeant dans du méthanol tout en conservant le vide et en agitant ;
4 - Ce cycle est répété jusqu'à ce qu'il n'y ait plus de bulles lors de la liquéfaction.

Ensuite le ballon laissé sous vide est placé à 130°C pendant 12 heures sous agitation magnétique. Après ces 12 heures, le mélange est solubilisé dans 100 g de chloroforme. Le cuivre présent dans le milieu est éliminé par filtration sur fritté puis par passage de la solution sur alumine.

Le polymère final est analysé en GPC et la $\overline{Mn}$ obtenue est égale à 92600 g/mole. L'analyse GPC montre que le pic correspondant au PABu de $\overline{Mn}$ = 8700 g/mole disparaît complètement lors de la copolymérisation avec le styrène pour donner le polymère triséquencé PS-PABu-PS, dont la séquence PABu a une $\overline{Mn}$ de 8700 g/mole, et les séquences de PS, une $\overline{Mn}$ de 45 000 g/mole.

EXEMPLE 2

On utilise comme macroamorceur le même PABu que celui obtenu à la première étape de polymérisation de l'Exemple 1 ($\overline{Mn}$ = 8700 g/mole). 1 g de ce polymère est mélangé à 2,05 g de styrène, à 60 mg de CuCl et à 0,277 g de bipyridine et on suit le même mode opératoire que celui de la deuxième étape de polymérisation de l'Exemple 1 ; le copolymère final a une $\overline{Mn}$ de 13000 g/mole, d'où un PS-PABu-PS à séquences PS de 2200 g/mole chacune.

EXEMPLE 3 : Préparation d'un polymère triséquencé PS-PABu-PS

(a) Synthèse de l'amorceur

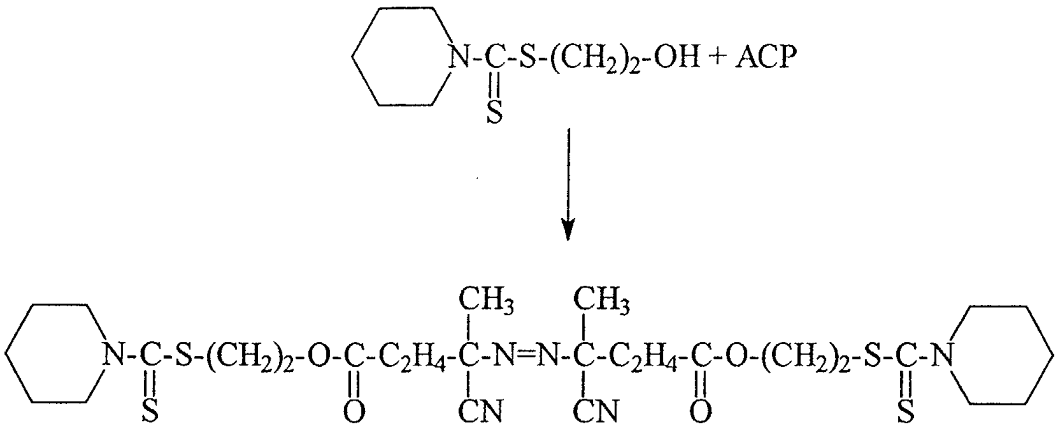

L'alcool de départ est formé par la réaction de substitution nucléophile entre le 1-chloro-2-hydroxyéthane et l'anion pipéridinodithiocarbamate (issu de la réaction d'addition entre le disulfure de carbone (1 mole) et la pipéridine (2 moles)). La substitution nucléophile est réalisée dans de l'éthanol absolu. Après avoir solubilisé 5 g de pipéridinodithiocarbamate dans 100 ml d'éthanol à 70°C, 2,5 g de 1-chloro-2-hydroxyéthane sont additionnés. Après addition complète, le mélange réactionnel est porté à reflux pendant 4 heures. Ensuite, le mélange réactionnel est lavé à l'eau à la température ambiante et le produit final est extrait au dichlorométhane. La phase organique est séchée avec du sulfate de magnésium, filtrée puis évaporée.

5 g d'acide 4,4'-azobis(4-cyanovalérique) et 0,7 g de l'alcool de départ sont solubilisés dans 100 ml de tétrahydrofuranne (THF) dans un ballon de 250 ml. Après avoir refroidi le mélange réactionnel à 0°C, la solution suivante (9,2 g de DCC, 0,25 g de DMAP et 20 ml de THF) est additionnée au goutte à goutte à l'aide d'une ampoule à brome. Une fois l'addition terminée, la réaction continue pendant environ 4 heures à la température ambiante.

Le mélange réactionnel est ensuite précipité à 0C dans du pentane afin d'éliminer l'acide 4,4'-azobis(4-cyanovalérique) résiduel. Le rendement est de 75%.

(b) Première étape de la polymérisation

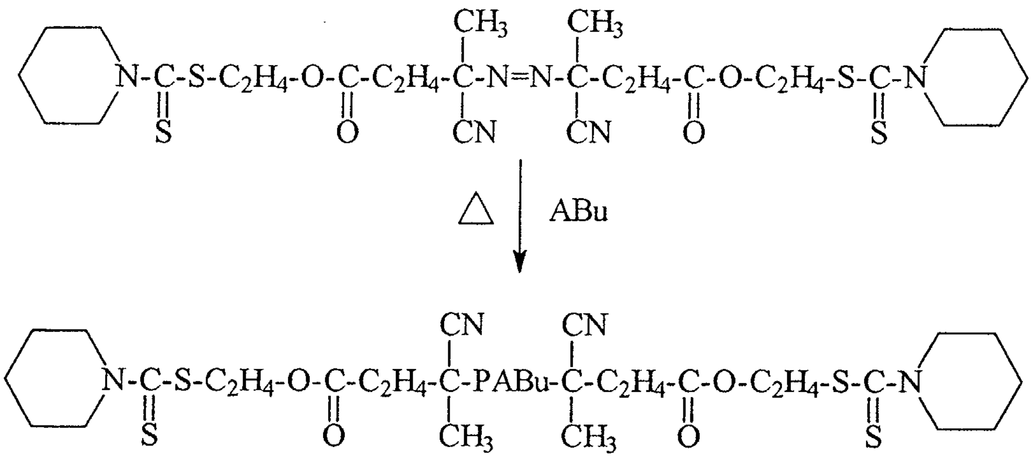


0,2 g de l'amorceur ainsi obtenu et 5 g d'ABu sont solubilisés dans 20 ml d'acétonitrile dans un ballon monocol

de 50 ml surmonté d'un réfrigérant. Après avoir placé le mélange réactionnel sous atmosphère inerte (trois cycles de purge vide azote), le mélange réactionnel est porté à 80°C pendant 5 heures.

Après élimination du solvant et du monomère résiduel par traitement thermique sous vide, la conversion du monomère est de 70% massique. Le PABu obtenu, analysé par GPC avec un étalonnage de polystyrène, se caractérise par une $\overline{Mn}$ = 66300 g/mole et un indice de polymolécularité de 2,5.

(c) Deuxième étape de la polymérisation

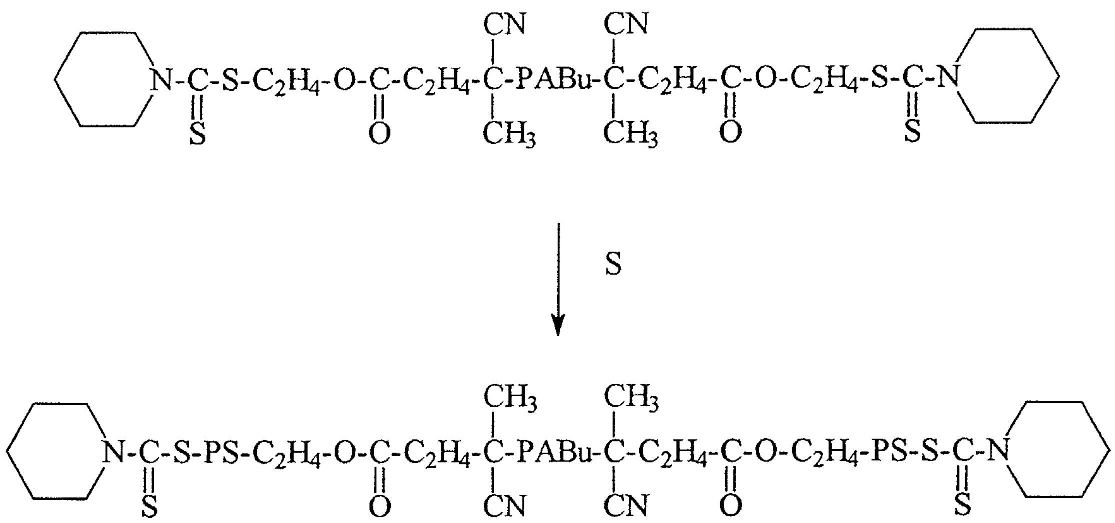


0,9 g du PABu précédemment synthétisé et caractérisé sont solubilisés dans 10 g de styrène dans un tube en verre de 20 ml. Le système, après avoir été mis sous atmosphère inerte (azote) est placé sous rayonnement UV (lampe Philips HPK 125W, 4A). La photopolymérisation est réalisée à 30°C avec une distance de 15 cm entre la lampe et le tube.

Après 6 heures de réaction sous UV, le mélange réactionnel est solubilisé dans 20 ml de THF et précipité dans 100 ml de méthanol. Après séchage sous vide, 2 g de polymère sont obtenus. La caractérisation RMN et GPC donnent respectivement un copolymère avec un % molaire de PABu de 65% et de styrène de 35% et une $\overline{Mn}$ = 150 000.

Ainsi le polymère final a une structure PS-PABu-PS avec une répartition de la $\overline{Mn}$ de 17 800-66 000-17 800, et un indice de polymolécularité de 2,4.

Exemple 4 : Préparation d'un copolymère biséquencé PMMA-PB

(a) Synthèse de l'amorceur

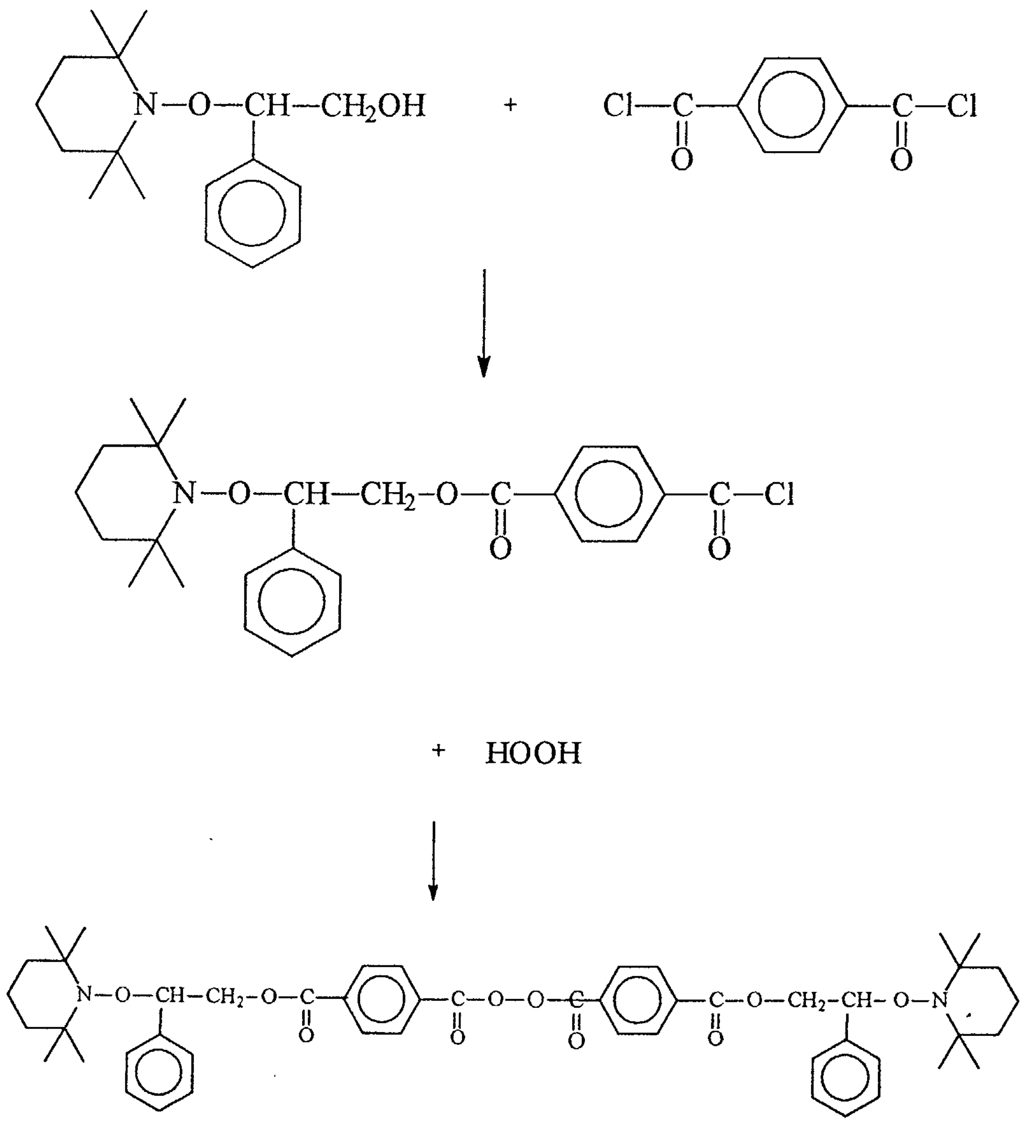

On synthétise l'alcool 1-(1-phényl-2-hydroxyéthoxy)-2,2,6,6-tétraméthylpyridine par une réaction de couplage entre un radical styryle (formé lors de l'étape d'amorçage entre un radical hydroxyle et une unité styrène) et une molécule de 2,2,6,6-tétraméthyl-1-pipéridinyloxy (TEMPO). Cette réaction est réalisée dans les conditions suivantes : 0,38 g (0,01 mole) de peroxyde d'hydrogène, 5,2 g (0,05 mole) de styrène et 3,12 g (0,02 mole) de 2,2,6,6-tétraméthyl-1-pipéridinyloxy (TEMPO) sont solubilisés dans 20 ml d'isopropanol. Le mélange est porté à reflux du solvant pendant 10 heures.

L'alcool formé est isolé par la technique de chromatographie sur colonne de silice avec un mélange éther de pétrole/éther (9/1) comme éluant. Le rendement est de 40%.

On prépare ensuite le précurseur du peroxyde fonctionnel. 5 g de l'alcool précédent (isolé et purifié) sont mis en solution dans du benzène (50 ml) en présence de 3,7 g de chlorure de téréphtaloyle, dans un réacteur de 100 ml surmonté d'un appareil DINSTARK. Le mélange réactionnel est porté à la température de l'azéotrope de mélange eau/benzène. Après 3 heures de réaction, le solvant est évaporé à l'évaporateur rotatif sous vide à température ambiante. Après purification par chromatographie sur colonne de silice avec un mélange éther de pétrole/éther (95:5) comme

éluant, au moins 30% de 1-chlorure 4-[1-(1-phényl-2-hydroxyéthoxy) -2,2,6, 6-tétraméthyl pipéridine)acide téréphtalique sont isolés.

Ensuite, 2 g du composé précédent sont dissous dans 5 ml de chloroforme et la solution est placée sous agitation magnétique à 0°C pendant 15 min. Parallèlement, dans 3 ml d'eau, 0,18 g de soude sont solubilisés et agités à 0°C. De même, 0,6 ml de peroxyde d'hydrogène à 20% sont placés à 0°C pendant 15 min. Ensuite, les deux dernières solutions sont ajoutées à la première préparation. Après 1 heure de réaction sous agitation magnétique à la température de 0°C, la phase chloroforme est récupérée. Après évaporation du chloroforme, une poudre blanche est obtenue, qui est ensuite recristallisée. Le rendement obtenu est au moins de 40%.

L'amorceur peroxyde synthétisé a la structure ci-dessus. A 80°C, le temps de demi-vie est de 3 x $10^{-5}s^{-1}$.

(b) Première étape de la polymérisation

0,1 g de l'amorceur peroxyde synthétisé ci-dessus est solubilisé dans 20 ml de toluène dans un réacteur en verre de 50 ml surmonté d'un réfrigérant. Ensuite, 2 g de MMA sont ajoutés et, après avoir placé le mélange réactionnel sous atmosphère inerte, le réacteur est porté à 90°C pendant 5 heures. Après avoir refroidi le mélange réactionnel à température ambiante, le mélange est purifié par précipitation dans 100 ml de méthanol. Après séchage du polymère, 1,4 g de PMMA sont recueillis, lequel, analysé par GPC, donne une masse moléculaire moyenne en nombre de 8200 g/mole et un indice de polymolécularité de 2,3.

(c) Deuxième étape de polymérisation

8 g de PMMA précédemment synthétisé sont placés dans un réacteur en inox de 100 ml en présence de 20 g de butadiène et 30 g de toluène. Le mélange réactionnel est porté à 130°C pendant 24 h. Après refroidissement et dégazage du mélange réactionnel, 10% de conversion en butadiène sont obtenus. Après caractérisation RMN du proton du polymère obtenu (calcul réalisé en prenant en compte les pics caractéristiques des doubles liaisons du polybutadiène comprises entre 5,4 et 5,9 ppm et le pic caractéristique des fonctions esters à 4 ppm du poly(méthacrylate de méthyle), les masses moléculaires relatives sont de 8200 g/mole pour le bloc PMMA et de 1600 g/mole pour le bloc de polybutadiène.

**Revendications**

**1.** Procédé de fabrication d'un polymère séquencé, **caractérisé par le fait que** :

- dans une première étape, on conduit une polymérisation radicalaire par voie thermique d'au moins un monomère $M_1$ à l'aide d'un amorceur de formule (I) :

$$X\text{-}A\text{-}Y \qquad (I)$$

dans laquelle :
- X et Y représentent chacun un groupement fonctionnel monovalent réactif et réactivable en polymérisation radicalaire vivante ;
- A représente un groupement divalent comprenant une liaison capable, en polymérisation radicalaire de se diviser pour générer des radicaux libres $X\text{-}D^\bullet$ et ${}^\bullet E\text{-}Y$, D et E étant les deux fragments de A issus de la division de ladite liaison,
pour obtenir un polymère pouvant être représenté par la formule (II) :

$$X\text{-}D\text{-}(\text{ou } Y\text{-}E\text{-})PM_1\text{-}T \qquad (II)$$

où T représente (1) un groupe terminal X-D- ou Y-E- qui se forme par recombinaison et/ou (2) un groupe terminal qui comporte une liaison insaturée et une liaison saturée et qui se forme par dismutation, les terminaisons T étant totalement ou essentiellement de type (2) dans le cas où $M_1$ est un monomère méthacrylique ;
- dans une seconde étape, on conduit une polymérisation radicalaire vivante du polymère (II) ainsi obtenu, conduisant à l'insertion entre les X et D et entre les Y et E de ce dernier d'une séquence polymère $PM_2$ d'au moins un monomère $M_2$, le copolymère vivant obtenu pouvant être utilisé comme macroamorceur dans une

nouvelle étape de polymérisation radicalaire vivante d'au moins un monomère $M_3$,
la polymérisation radicalaire vivante étant une polymérisation à activation photochimique en présence de radicaux libres.

**2.** Procédé selon la revendication 1, **caractérisé par le fait que** l'on choisit les monomères $M_1$, $M_2$ et le cas échéant $M_3$, parmi les monomères vinyliques, allyliques, vinylidéniques, diéniques et oléfiniques.

**3.** Procédé selon l'une des revendications 1 et 2, **caractérisé par le fait que** A comporte un groupement choisi parmi les groupements -N=N-, -O-O-,

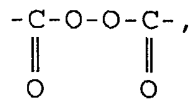

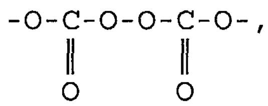 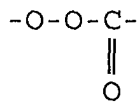 et 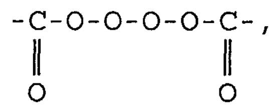

dont le
clivage fournit les radicaux libres en polymérisation radicalaire.

**4.** Procédé selon l'une des revendications 1 à 3, **caractérisé par le fait que** l'amorceur (I) est issu de la réaction de l'acide 4,4'-azobis(4-cyano-pentanoïque) avec un alcool portant un groupement fonctionnel X ou Y.

**5.** Procédé selon l'une des revendications 1 à 3, **caractérisé par le fait que** l'amorceur (I) est issu de la réaction entre le peroxyde d'hydrogène et un chlorure d'acide portant un groupement X ou Y.

**6.** Procédé selon l'une des revendications 1 à 5, **caractérisé par le fait que** l'on conduit la polymérisation de la première étape à une température comprise dans une plage allant de 0°C à une température inférieure à la température de dissociation des groupements X et Y.

**7.** Procédé selon l'une des revendications 1 à 6, **caractérisé par le fait que** l'activation photochimique a lieu en présence de radicaux libres provenant de groupements X et Y de formule (IV) :

$$R^{20}\text{-S-} \qquad \text{(IV)}$$

où $R^{20}$ est choisi parmi les radicaux alkyle, aryle, aralkyle et alkaryle, ayant par exemple 1 à 30 atomes de carbone et pouvant comporter des substituants ; les radicaux des formules :

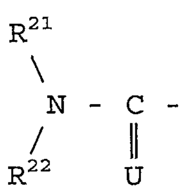 ; 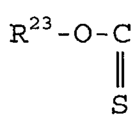 ;

dans lesquels U représente O ou S, et $R^{21}$, $R^{22}$ et $R^{23}$ sont choisis chacun parmi les radicaux alkyle, aryle, aralkyle et alkaryle, ayant par exemple de 1 à 30 atomes de carbone et pouvant comporter des substituants, $R^{21}$ et $R^{22}$ pouvant former, conjointement avec l'atome d'azote qui les porte, un cycle azoté en $C_4$-$C_{10}$, pouvant comporter des substituants.

**8.** Procédé selon la revendication 7, **caractérisé par le fait que** X et Y sont choisis parmi :

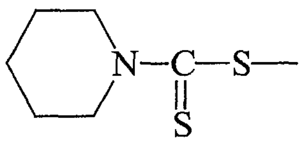 ; 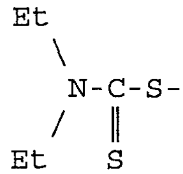 ;

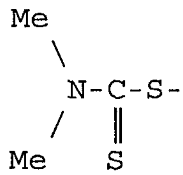 ; 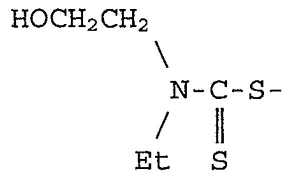 ;

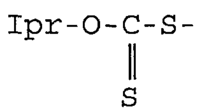

et le radical N-(2-phtalimido éthyl)pipérazine thiurame.

**8.** Copolymères séquencés susceptibles d'être obtenus par le procédé tel que défini à l'une des revendications 1 à 7.

**9.** Composé de formule :

$$X - L_1 - A' - L_2 - Y \quad (V)$$

dans laquelle :

- A' représente un groupement choisi parmi les groupements: -N=N-, -O-O-,

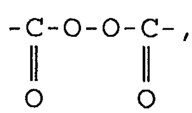, 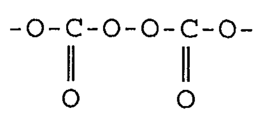,

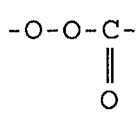 et 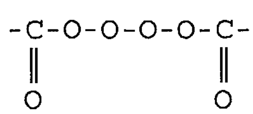 ;

- $L_1$ et $L_2$ représentent chacun un groupement de liaison divalent ; et
- X et Y représentent chacun un groupement choisi parmi ceux de la formule (IV) :

$$R^{20}\text{-S-} \quad (IV)$$

où $R^{20}$ est choisi parmi les radicaux alkyle, aryle, aralkyle et alkaryle, ayant par exemple 1 à 30 atomes de carbone et pouvant comporter des substituants ; les radicaux des formules :

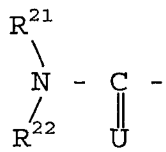 ; 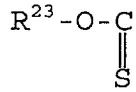 ;

dans lesquels U représente O ou S, et $R^{21}$, $R^{22}$ et $R^{23}$ sont choisis chacun parmi les radicaux alkyle, aryle, aralkyle et alkaryle, ayant par exemple de 1 à 30 atomes de carbone et pouvant comporter des substituants, $R^{21}$ et $R^{22}$ pouvant former, conjointement avec l'atome d'azote qui les porte, un cycle azoté en $C_4$-$C_{10}$, pouvant comporter des substituants.

**10.** Composé selon la revendication 9, de formule :